(19) Europäisches Patentamt / European Patent Office / Office européen des brevets

(11) **EP 4 362 763 B1**

(12) **FASCICULE DE BREVET EUROPEEN**

(45) Date de publication et mention de la délivrance du brevet:
**23.04.2025 Bulletin 2025/17**

(21) Numéro de dépôt: **22738738.8**

(22) Date de dépôt: **20.06.2022**

(51) Classification Internationale des Brevets (IPC):
***A61B 1/00*** (2006.01)

(52) Classification Coopérative des Brevets (CPC):
**A61B 1/00009; A61B 1/00165**

(86) Numéro de dépôt international:
**PCT/IB2022/055698**

(87) Numéro de publication internationale:
**WO 2023/275664 (05.01.2023 Gazette 2023/01)**

(54) **PROCÉDÉ D'IMAGERIE, ENDOSCOPE ET PRODUIT PROGRAMME D'ORDINATEUR**

BILDGEBUNGSVERFAHREN, ENDOSKOP UND COMPUTERPROGRAMMPRODUKT

IMAGING METHOD, ENDOSCOPE AND COMPUTER PROGRAM PRODUCT

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **01.07.2021 FR 2107170**

(43) Date de publication de la demande:
**08.05.2024 Bulletin 2024/19**

(73) Titulaires:
• **Université Grenoble Alpes
38400 Saint-Martin-d'Hères (FR)**
• **Centre National de la Recherche Scientifique
75794 Paris Cedex 16 (FR)**

(72) Inventeurs:
• **BOSSY, Emmanuel
38400 Saint-Martin-d'Hères (FR)**
• **CARAVACA AGUIRRE, Antonio Miguel
38400 Saint-Martin-d'Hères (FR)**

(74) Mandataire: **Novaimo
Europa 1
362, avenue Marie Curie
Archamps Technopole
74166 Saint-Julien-en-Genevois Cedex (FR)**

(56) Documents cités:
US-A1- 2015 377 701     US-B2- 10 398 294
US-B2- 10 809 750       US-B2- 6 894 828
US-B2- 9 661 986

**Description**

La présente invention concerne le domaine technique de l'imagerie, et porte plus particulièrement sur un procédé d'imagerie et sur un endoscope mettant en œuvre le procédé d'imagerie.

Actuellement, les plupart des endoscopes utilisent un faisceau de fibres monomodes pour réaliser une image d'un objet à imager placé en sortie du faisceau de fibres : on allume fibre par fibre du faisceau de fibres en balayant régulièrement le faisceau de fibres, puis on recueille pour chaque fibre la lumière reçue de l'objet à imager pour déduire une image de l'objet à imager pixel par pixel.

Les résultats d'imagerie obtenus par ces endoscopes existants sont bons. Cependant ces endoscopes de l'état de la technique présentent des inconvénients.

Essentiellement, le faisceau de fibres monomodes a un diamètre important, ce qui rend les endoscopes de l'état de la technique difficilement utilisables dans des procédures où la sonde d'imagerie de l'endoscope, constituée par le faisceau de fibres monomodes, doit être invasive de manière minimale.

Dans la présente demande, en accord avec l'usage courant, une fibre optique multimode est définie comme étant une fibre avec un unique cœur de grand diamètre, qui permet à plusieurs modes de se propager, par opposition à une fibre optique monomode ayant un unique cœur dans lequel un unique mode peut se propager. Il est à noter qu'il faut distinguer une fibre optique multimode d'une fibre optique multicoeurs, qui comprend plusieurs cœurs, chaque cœur étant en général monomode.

L'imagerie optique dans des fibres optiques multimodes est un domaine de recherche qui se développe depuis une dizaine d'années. En particulier, l'imagerie optique endoscopique à travers des fibres optiques multimodes permet de réaliser des systèmes d'imagerie endoscopique de dimensions très réduites par rapport à l'approche classique basée sur des faisceaux (bundles) de fibres optiques monomodes. On utilise généralement des fibres optiques multimodes standards (à section circulaire), sans composant optique du côté échantillon (terminologie "lensless endoscope") et une calibration préalable est nécessaire pour la réalisation d'une image, en raison du fait qu'une fibre optique multimode est un milieu de propagation complexe. La demande de brevet américain US2015/0015879A1 et les brevets américains US5956447 et US10254534B2 divulguent de tels exemples d'endoscopes à fibre multimode. Ces endoscopes peuvent être utilisés dans des procédures où la sonde d'imagerie de l'endoscope est minimalement invasive.

US10809750B2 et US10398294B2 décrivent également des endoscopes selon l'état de la technique.

Cependant, dans la fibre multimode, du fait de l'utilisation d'un seul milieu pour propager plusieurs modes, l'information est mélangée/brouillée au cours de la propagation, la compensation de ce brouillage ne pouvant se faire que par l'intermédiaire d'un calibrage préalable rendant l'utilisation de tels endoscopes à fibre multimode longue et peu conviviale.

En outre, le calibrage dépend de la forme de la fibre optique multimode. Si la forme de la fibre optique multimode change après le calibrage, le calibrage est à refaire, et ce même pour des changements de forme très faible. Pour pallier les effets du changement de forme de la fibre optique multimode, on utilise donc des fibres optiques multimodes rigides, ce qui limite fortement les domaines d'application et l'utilisation pratique de l'endoscope.

La possibilité d'imager dans des fibres flexibles n'a été démontrée qu'avec un type particulier de fibres optiques, appelé fibres multi-cœur, pour lesquelles la phase de calibration reste approximativement valable même après un changement de conformation de la fibre.

Mais ces fibres sont similaires dans leur principe aux faisceaux (bundles) de fibres monomodes, et de ce fait présentent le même inconvénient en termes de taille, à savoir d'avoir une section typiquement 20 à 30 fois supérieure aux fibres optiques multimodes de l'invention.

Il existe donc un besoin pour un endoscope minimalement invasif, sans étape de calibration préalable, pouvant permettre l'utilisation d'une fibre optique souple, quelle que soit sa configuration (droite ou courbée) pour un plus grand confort d'utilisation de l'endoscope, que l'invention se propose de résoudre avec l'utilisation d'une fibre optique multimode à section carrée. Dans la présente demande, en accord avec l'usage courant, une fibre optique multimode à section carrée est définie comme une fibre optique multimode dont le cœur est à section carrée.

Il existe dans ces fibres optiques multimodes à section carrée un effet mémoire de translation permettant de s'affranchir de la phase de calibration. En effet, il a été démontré qu'un effet mémoire présent dans le cas de milieux diffusants permet d'imager un objet pour l'imagerie sans calibration préalable du milieu diffusant. Notons que cela suppose un effet mémoire dans toutes les directions. Dans les fibres optiques multimodes à section circulaire, un effet mémoire de rotation (donc dans une direction seulement) a été décrit, ce qui est insuffisant pour réaliser une image.

Le procédé de l'invention pour former une image de l'objet à imager repose sur deux étapes, 1) une étape de mesure, et 2) une étape de reconstruction de l'objet. Sa force repose sur le fait qu'il n'y a pas d'étapes de calibration, et qu'elle s'applique même quand la fibre est courbée.

Les inventeurs ont montré que le champ optique en sortie de la fibre optique multimode à section carrée se décompose en la somme de quatre champs optiques, qui tous translatent quand on translate le motif d'éclairage en entrée. Ce "quadruple effet mémoire" est une généralisation de l'effet mémoire bien connu dans la communauté, qui correspond à la translation d'un unique champ de sortie quand le champs d'entrée translate.

Le cœur de l'invention repose sur l'existence du "quadruple" effet mémoire dans les fibres optiques multimodes à section carrée, pour la reconstruction sans calibration. De plus, cet effet a été observé même quand la fibre optique multimode à section carrée est courbée, ce qui rend le procédé de l'invention applicable à des fibres flexibles (à condition que la fibre ne bouge pas pendant la mesure).

La présente invention a donc pour objet un procédé d'imagerie d'un objet à imager, caractérisé par le fait qu'il comprend :

- générer, par un dispositif d'éclairage, une série de motifs d'éclairage ;
- pour chaque motif d'éclairage, réaliser une phase de stimulation dans laquelle des translations du motif d'éclairage sont réalisées en entrée d'une fibre optique multimode à section carrée au moyen d'au moins un dispositif de translation d'un système optique disposé entre le dispositif d'éclairage et l'entrée de la fibre optique multimode à section carrée, les translations étant réalisées dans un plan parallèle au plan d'entrée de la fibre optique multimode à section carrée ;
- pour chaque phase de stimulation, réaliser une phase d'acquisition dans laquelle le signal généré par le motif d'éclairage sur l'objet à imager placé à la sortie de la fibre optique multimode à section carrée est mesuré par un dispositif de mesure de signal ; et
- reconstruire par un dispositif de calcul, au moyen du signal mesuré pour chacune des phases d'acquisition, l'objet à imager.

L'invention repose sur l'utilisation de fibres optiques multimodes à section carrée. Contrairement aux fibres utilisées classiquement, qui sont à section circulaire, les inventeurs ont démontré théoriquement et expérimentalement qu'il existe dans ces fibres optiques multimodes à section carrée une propriété d'invariance de translation (appelée dans la littérature effet mémoire de translation : le motif en sortie du milieu (ici la fibre optique multimode à section carrée) translate quand on translation le motif d'éclairage), effet qui n'existe pas dans les fibres optiques multimodes à section circulaire. L'exploitation de cet effet mémoire dans les fibres optiques multimodes à section carrée permet de s'affranchir de la phase de calibration (totalement ou partiellement), et permet notamment de réaliser des images dans des fibres souples (dont la forme peut être modifiée en cours d'utilisation), deux limites des méthodes actuelles reposant sur les fibres standards à section circulaire, qui nécessitent une calibration et qui ne fonctionnent que pour une forme figée de la fibre. Les fibres optiques multimodes à section carrée sont un produit commercial existant, utilisées à des fins de contrôle de la forme d'un motif d'éclairage. L'invention concerne l'utilisation totalement nouvelle de ces fibres pour l'imagerie optique endoscopique. La fibre optique multimode à section carrée permet de réduire grandement le diamètre de la fibre par rapport aux endoscopes actuels à faisceaux de fibres monomodes, permettant d'avoir une section de la fibre optique multimode à section carrée typiquement 20 à 30 fois inférieure aux faisceaux de fibres monomodes de l'état de la technique, et donc contribue à diminuer le caractère intrusif de l'endoscope pour obtenir un dispositif miniature.

Aucune optique n'est présente en sortie de la fibre optique multimode à section carrée, entre la sortie de la fibre optique multimode à section carrée et l'objet à imager, ce qui rend la structure en sortie de fibre extrêmement simple et robuste.

Le nombre de motifs d'éclairage dans la série doit être supérieur ou égal à un. Cependant, plus le nombre de motifs d'éclairage dans la série est élevé, meilleure est la reconstruction de l'objet à imager.

Le motif d'éclairage est de préférence un motif de tavelures (en anglais un motif de speckle) mais peut également être un spot focalisé, sans s'éloigner du cadre de la présente invention.

Le système optique peut comprendre un ou plusieurs composants optiques tels que lentilles, miroirs, réseaux, prismes, diffuseurs, séparateurs, diaphragmes, lames à retard, polariseurs, filtres, diviseurs de faisceaux, expanseurs de faisceaux, concentrateurs, matrices de micro-miroirs, etc...

Selon un mode de réalisation, l'au moins un dispositif de translation est l'un parmi un modulateur spatial de lumière à base de cristaux liquides, un modulateur spatial de lumière de type matrice de micro-miroirs (ou DMD, acronyme de l'anglais Digital Micromirror Device), un diffuseur optique associé à une platine de translation, un diffuseur optique associé à des miroirs galvanométriques. Les diffuseurs optiques peuvent notamment être un verre diffusant.

Selon un mode de réalisation, le dispositif de mesure de signal est un capteur optique de mesure du signal optique réfléchi par l'objet à imager associé à une fibre optique de détection placée entre l'objet à imager et le capteur optique, la fibre optique de détection étant l'une parmi la fibre optique multimode à section carrée et une fibre optique auxiliaire. Un diviseur de faisceau est présent dans le système optique pour transférer le signal optique généré par le motif d'éclairage sur l'objet à imager dans la fibre optique multimode à section carrée vers le capteur optique.

Selon un mode de réalisation, le dispositif de mesure de signal est un hydrophone à fibre optique mesurant le signal photoacoustique de l'objet à imager. La fibre optique de l'hydrophone est alors reliée à un dispositif de détection photoacoustique par son extrémité opposée à celle en regard de l'objet à imager.

Selon un mode de réalisation, l'objet à imager est fluorescent et le dispositif de mesure de signal est un capteur optique de mesure du signal de fluorescence émis par l'objet à imager associé à une fibre optique de détection placée entre l'objet à imager et le capteur optique, la fibre optique de détection étant l'une parmi la fibre optique multimode à section carrée et une fibre optique auxiliaire. Un filtre dichroïque est alors présent dans le système optique pour filtrer le signal optique généré par le motif d'éclairage sur l'objet à imager dans la fibre optique multimode à section carrée ou la fibre optique auxiliaire, lequel signal filtré est ensuite envoyé par le système optique vers le capteur optique.

Selon un mode de réalisation, dans chaque phase de stimulation, les translations sont réalisées dans tout ou partie du plan d'entrée de la fibre optique multimode à section carrée.

Selon un mode de réalisation, le dispositif d'éclairage est une source de lumière cohérente. Le dispositif d'éclairage doit en effet conduire à une figure de tavelures (speckle en anglais) en sortie de la fibre optique multimode à section carrée. Le dispositif d'éclairage sera de préférence monochromatique, et de façon davantage préférée un laser.

Selon un mode de réalisation, le dispositif de calcul est sélectionné dans au moins l'un parmi un ordinateur, un microprocesseur, un processeur de signaux numériques, DSP, un processeur, un microcontrôleur, une matrice prédiffusée programmable, FPGA, un composant à application spécifique, ASIC, et comprend de la mémoire.

Selon un mode de réalisation, la reconstruction de l'objet à imager par le dispositif de calcul comprend une première étape comprenant, pour chaque phase d'acquisition, le calcul de l'autocorrélation spatiale du signal mesuré puis le calcul de l'autocorrélation moyennée pour tous les motifs d'éclairage, et une seconde étape de déduction de l'objet à imager à partir de l'autocorrélation moyennée pour tous les motifs d'éclairage obtenue dans la première étape.

Selon un mode de réalisation, l'objet à imager est déduit de l'autocorrélation moyennée pour tous les motifs d'éclairage par résolution de l'équation suivante :

[Math.1]

$$\hat{O}_{estim\acute{e}}(\mathbf{r}) = argmin_X \, \| \, C^S_{mesure}(\Delta \mathbf{r}_{in}) - \iint C^I(\Delta \mathbf{r}_{in}, \, \Delta \mathbf{r}_{out}) C^X(\Delta \mathbf{r}_{out}) \mathrm{d}^2 \Delta \mathbf{r}_{out} \, \|$$

où $\Delta \mathbf{r}_{in} = (\Delta x_{in}, \Delta y_{in})$ définit une position de balayage dans la face d'entrée de la fibre optique multimode à section carrée avec $x_{in}$ et $y_{in}$ les coordonnées dans la face d'entrée de la fibre optique multimode à section carrée, $\Delta \mathbf{r}_{out}$ représente les coordonnées de la face de sortie de la fibre optique multimode à section carrée, $C^I$ est l'autocorrélation moyenne des N motifs d'éclairage, $C^X$ est l'autocorrélation d'un objet test X, $C^S_{mesure}$ est l'autocorrélation moyennée pour tous les motifs d'éclairage et $\hat{O}_{estim\acute{e}}(\mathbf{r})$ est l'estimation obtenue de l'objet (O) à imager.

Selon un mode de réalisation, l'objet à imager est déduit de l'autocorrélation moyennée pour tous les motifs d'éclairage par un réseau de neurones préalablement entraîné sur une classe d'objets définie, le réseau de neurones fournissant une estimation de l'objet à imager à partir de l'autocorrélation moyennée, par comparaison de l'autocorrélation moyennée connue par le réseau de neurones sur les objets de la classe définie à l'autocorrélation moyennée calculée pour l'objet à image.

Un réseau de neurones de type U-NET peut par exemple être utilisé comme réseau de neurones. La méthode utilisée est par exemple décrite dans la publication scientifique « Compensating for visibility artefacts in photoacoustic imaging with a deep learning approach providing prediction uncertainties » (Compensation d'artéfacts de visibilité en imagerie photoacoustique avec une approche apprentissage profond fournissant des incertitudes de prédiction), Guillaume Godefroy, Bastien Arnal, Emmanuel Bossy, Photoacoustics, 21 :100218, 2021. 4.

L'ensemble de données d'entraînement pour le réseau de neurones avec cette méthode est constitué par d'une série d'objets test connus et de leur fonction d'autocorrélation moyenne (connue également). Les objets de la série doivent appartenir à une classe d'objets définie (par exemple une série de caractères manuscrits, des populations de cellules, des vaisseaux sanguins), et le réseau permettra de reconstruire un objet inconnu sous réserve qu'il fasse partie de la famille à partir de laquelle le réseau a été entraîné. Un exemple de classe d'objets connus très utilisée est donnée par la base de données MNIST de chiffres manuscrits (https://en.wikipedia.org/wiki/MNIST_database).

Selon un mode de réalisation, la fibre optique multimode à section carrée est souple. L'invention permet ainsi une imagerie avec une fibre souple, avec possibilité que la forme de la fibre optique multimode à section carrée change entre deux mesures, pour autant qu'elle ne change pas pendant une mesure donnée, permettant plus de souplesse pour le procédé d'imagerie et donc une plus grande facilité d'utilisation.

Selon un mode de réalisation, la fibre optique multimode à section carrée comprend une gaine recouvrant une âme de section carrée, l'âme ayant des dimensions comprises entre 10 μm*10 μm et 1 mm*1 mm, de façon préférée comprises entre 50 μm*50 μm et 200 μm*200 μm, de façon davantage préférée comprise entre 100 μm*100 μm et 150 μm*150 μm. L'objectif de miniaturisation de l'invention est ainsi atteint avec des diamètres de fibres 20 à 30 fois inférieurs aux faisceaux de fibres monomodes de l'état de la technique.

L'invention a également pour objet un endoscope pour l'imagerie d'un objet à imager, caractérisé par le fait qu'il comprend un dispositif d'éclairage, un système optique, au moins une fibre optique multimode à section carrée, le système optique couplant optiquement le dispositif d'éclairage à l'entrée de l'au moins une fibre optique multimode à section carrée, l'objet à imager étant configuré pour être placé en sortie de l'au moins une fibre optique multimode à section carrée, un dispositif de mesure de signal généré par l'objet à imager et un dispositif de calcul pour mettre en œuvre le procédé tel que décrit ci-dessus.

Etant donné le diamètre de la fibre optique multimode à section carré, l'endoscope selon l'invention est ainsi minimalement invasif et ne nécessite aucune phase préalable de calibrage, réduisant grandement le temps d'imagerie.

Selon un mode de réalisation, le système optique comprend un dispositif de translation pour translater un motif d'éclairage généré par le dispositif d'éclairage sur tout ou partie du plan d'entrée de l'au moins une fibre optique multimode à section carrée, le dispositif de translation étant configuré pour translater le motif d'éclairage dans un plan parallèle au plan d'entrée de l'au moins fibre optique multimode à section carrée, le dispositif de translation étant l'un parmi un modulateur spatial de lumière à base de cristaux liquides, un modulateur spatial de lumière de type matrice de micro-miroirs (DMD), un diffuseur optique associé à une platine de translation, un diffuseur optique associé à des miroirs galvanométriques. Les diffuseurs optiques peuvent notamment être un verre diffusant.

Selon un mode de réalisation, le dispositif de mesure de signal est constitué par au moins l'un parmi :

- l'au moins une fibre optique multimode à section carrée associée à un capteur optique en entrée de la fibre optique multimode à section carrée pour capter un signal optique réfléchi par l'objet à imager, le système optique comprenant alors un diviseur de faisceau pour envoyer le signal généré par l'objet à travers l'au moins une fibre optique multimode à section carrée vers le capteur optique,
- au moins une fibre optique auxiliaire associée à un capteur optique du côté entrée de la fibre optique multimode à section carrée de l'endoscope pour capter un signal optique réfléchi par l'objet à imager,
- un hydrophone à fibre optique pour capter le signal photoacoustique de l'objet à imager, associé à un dispositif de détection photoacoustique.

L'au moins une fibre optique multimode à section carrée sera de préférence accolée, le cas échéant, à la fibre optique auxiliaire ou à la fibre optique d'hydrophone à des fins de réduction d'encombrement pour rendre le caractère intrusif minimal pour l'extrémité de l'endoscope.

Selon un mode de réalisation, lorsque l'objet à imager est fluorescent, le dispositif de mesure de signal est constitué par au moins l'un parmi :

- l'au moins une fibre optique multimode à section carrée associée à un capteur optique en entrée de la fibre optique multimode à section carrée pour capter un signal de fluorescence de l'objet à imager, le système optique comprenant alors un filtre dichroïque pour envoyer le signal généré par l'objet à travers l'au moins une fibre optique multimode à section carrée vers le capteur optique,
- au moins une fibre optique auxiliaire associée à un capteur du côté entrée de la fibre optique multimode à section carrée de l'endoscope pour capter un signal de fluorescence de l'objet à imager, le système optique comprenant alors un filtre dichroïque pour envoyer le signal généré par l'objet à travers l'au moins une fibre optique auxiliaire vers le capteur optique.

Selon un mode de réalisation, le dispositif de calcul est sélectionné dans au moins l'un parmi un ordinateur, un microprocesseur, un processeur de signaux numériques, DSP, un processeur, un microcontrôleur, une matrice prédiffusée programmable, FPGA, un composant à application spécifique, ASIC, et comprend de la mémoire. Le dispositif de calcul peut faire partie de l'endoscope selon l'invention ou être déporté et connecté à l'endoscope.

Selon un mode de réalisation, l'au moins une fibre multimode à section carrée est souple. Il est ainsi possible d'avoir un endoscope souple.

Selon un mode de réalisation, l'au moins une fibre multimode à section carrée comprend une gaine recouvrant une âme de section carrée, l'âme ayant des dimensions comprises entre 10 $\mu$m*10 $\mu$m et 1 mm*1 mm, de façon préférée comprises entre 50 $\mu$m*50 $\mu$m et 200 $\mu$m*200 $\mu$m, de façon davantage préférée comprise entre 100 $\mu$m*100 $\mu$m et 150 $\mu$m*150 $\mu$m.

L'invention a également pour objet un produit programme d'ordinateur, caractérisé par le fait qu'il comprend des instructions qui, lorsqu'elles sont chargées et exécutées sur un dispositif de calcul d'un endoscope tel que décrit ci-dessus, permettent de mettre en œuvre le procédé tel que décrit ci-dessus.

Pour mieux illustrer l'objet de la présente invention, des modes de réalisation particulier de celle-ci vont maintenant être décrits, en liaison avec les dessins annexés.

Sur ces dessins :

[Fig.1] représente un endoscope selon un premier mode de réalisation de l'invention ;

[Fig.2] représente un endoscope selon un deuxième mode de réalisation de l'invention ;

[Fig.3] représente un endoscope selon un troisième mode de réalisation de l'invention ;

[Fig.4] représente schématiquement une fibre multimode à section carrée selon l'invention ; et

[Fig.5] représente schématiquement les étapes du procédé d'imagerie selon l'invention.

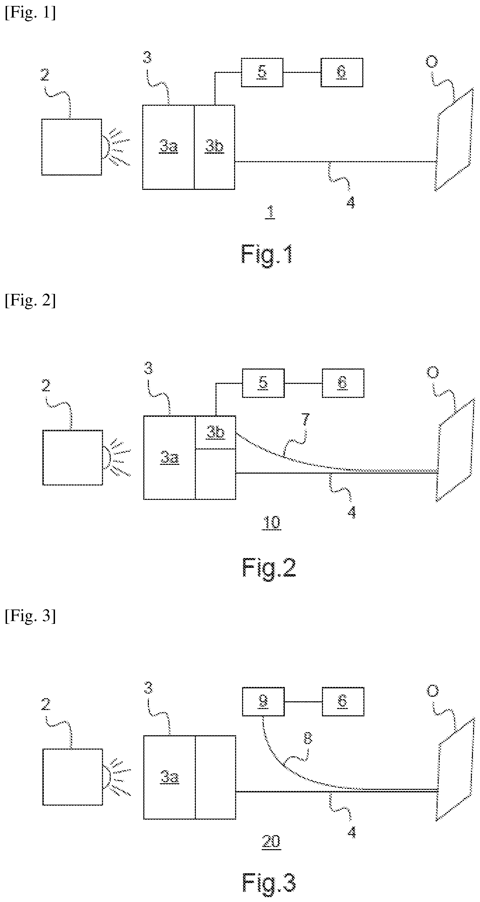

Si l'on se réfère à la [Fig.1], on peut voir que l'on a représenté un endoscope 1 selon un premier mode de réalisation de l'invention.

L'endoscope 1 comprend un dispositif d'éclairage 2, générant une lumière cohérente, de préférence monochromatique, de préférence un laser.

En regard du dispositif d'éclairage 2 dans l'endoscope 1 est disposé un système optique 3 qui envoie la lumière générée par le dispositif d'éclairage 2 dans une fibre optique multimode à section carrée 4. La sortie de la fibre optique multimode à section carrée 4 débouche directement (sans élément optique) sur un objet à imager O. Un dispositif de mesure 5 est relié au système optique 3 et à un dispositif de calcul 6.

Le système optique 3 comprend un dispositif de translation 3a pour translater un motif d'éclairage généré par le dispositif d'éclairage 2 sur tout ou partie du plan d'entrée de l'au moins une fibre optique multimode à section carrée 4. Le dispositif de translation 3a peut être l'un parmi un modulateur spatial de lumière à base de cristaux liquides, un modulateur spatial de lumière de type matrice de micro-miroirs, un diffuseur optique associé à une platine de translation, un diffuseur optique associé à des miroirs galvanométriques.

Le système optique 3 comprend également un diviseur de faisceau 3b, permettant de rediriger le faisceau optique généré par l'éclairage de l'objet à imager O dans la fibre optique multimode à section carrée 4 vers le dispositif de mesure 5.

Dans ce premier mode de réalisation, le dispositif d'éclairage 2 génère un motif d'éclairage dirigé vers le système optique 3, à l'intérieur duquel le dispositif de translation 3a translate le motif d'éclairage sur la face d'entrée de la fibre optique multimode à section carrée 4, laquelle conduit ce motif d'éclairage vers l'objet à imager O, qui renvoie dans la fibre optique multimode à section carrée 4 un signal optique réfléchi, transmis par l'intermédiaire du diviseur de faisceau 3b vers le dispositif de mesure 5 lequel, associé au dispositif de calcul 6 permet de reconstruire l'objet à imager O avec la méthode décrite ci-après plus en détail.

Avec ce premier mode de réalisation, il est possible de capter par la fibre optique multimode à section carrée 4 soit le signal optique réfléchi, soit une fluorescence générée par l'objet à imager O, le dispositif de mesure étant dans les deux cas un capteur optique.

Si l'on se réfère à la [Fig.2], on peut voir que l'on a représenté un endoscope 1 selon un deuxième mode de réalisation de l'invention.

Comme pour le premier mode de réalisation, l'endoscope 10 comprend un dispositif d'éclairage 2, générant une lumière cohérente, de préférence monochromatique, de préférence un laser.

En regard du dispositif d'éclairage 2 dans l'endoscope 10 est disposé un système optique 3 qui envoie la lumière générée par le dispositif d'éclairage 2 dans une fibre optique multimode à section carrée 4. La sortie de la fibre optique multimode à section carrée 4 débouche directement (sans élément optique) sur un objet à imager O. Un dispositif de mesure 5 est relié au système optique 3 et à un dispositif de calcul 6.

Une fibre optique auxiliaire 7 capte le signal optique réfléchi généré par l'objet à imager O, et le renvoie vers le diviseur de faisceau 3b du système optique 3.

Dans ce deuxième mode de réalisation, le dispositif d'éclairage 2 génère un motif d'éclairage dirigé vers le système optique 3, à l'intérieur duquel le dispositif de translation 3a translate le motif d'éclairage sur la face d'entrée de la fibre optique multimode à section carrée 4, laquelle conduit ce motif d'éclairage vers l'objet à imager O, qui renvoie dans la fibre optique auxiliaire 7 un signal optique réfléchi, transmis par l'intermédiaire du diviseur de faisceau 3b vers le dispositif de mesure 5 lequel, associé au dispositif de calcul 6 permet de reconstruire l'objet à imager O avec la méthode décrite ci-après plus en détail.

Avec ce deuxième mode de réalisation, il est possible de capter par la fibre optique auxiliaire 7 soit la lumière réfléchie, auquel cas le dispositif de mesure 5 est un capteur optique de lumière réfléchie (avec la même longueur d'onde), soit une fluorescence générée par l'objet à imager O, auquel cas le dispositif de mesure est un capteur optique de fluorescence.

Si l'on se réfère à la [Fig.3], on peut voir que l'on a représenté un endoscope 1 selon un troisième mode de réalisation de l'invention.

Comme pour le premier mode de réalisation, l'endoscope 20 comprend un dispositif d'éclairage 2, générant une lumière cohérente, de préférence monochromatique, de préférence un laser.

En regard du dispositif d'éclairage 2 dans l'endoscope 20 est disposé un système optique 3 qui envoie la lumière générée par le dispositif d'éclairage 2 dans une fibre optique multimode à section carrée 4. La sortie de la fibre optique multimode à section carrée 4 débouche directement (sans élément optique) sur un objet à imager O. Un hydrophone 9 à fibre optique 8 est relié au système optique 3 et à un dispositif de calcul 6.

La fibre optique 8 de l'hydrophone 9 capte un signal photoacoustique généré par l'objet à imager O, et le renvoie vers l'hydrophone 9, lequel hydrophone 9 envoie un signal vers le dispositif de calcul 6.

Dans ce troisième mode de réalisation, le dispositif d'éclairage 2 génère un motif d'éclairage dirigé vers le système optique 3, à l'intérieur duquel le dispositif de translation 3a translate le motif d'éclairage sur la face d'entrée de la fibre optique multimode à section carrée 4, laquelle conduit ce motif d'éclairage vers l'objet à imager O, qui renvoie dans la fibre optique 8 un signal photoacoustique, transmis par l'intermédiaire de l'hydrophone 9 vers le dispositif de calcul 6 pour reconstruire l'objet à imager O avec la méthode décrite ci-après plus en détail.

Avec ce troisième mode de réalisation, on peut capter un signal photoacoustique généré par l'objet à imager O.

Dans les trois modes de réalisation, le dispositif de calcul 6 est au moins l'un parmi un ordinateur, un microprocesseur, un processeur de signaux numériques, DSP, un processeur, un microcontrôleur, une matrice prédiffusée programmable, FPGA, un composant à application spécifique, ASIC, et comprend de la mémoire

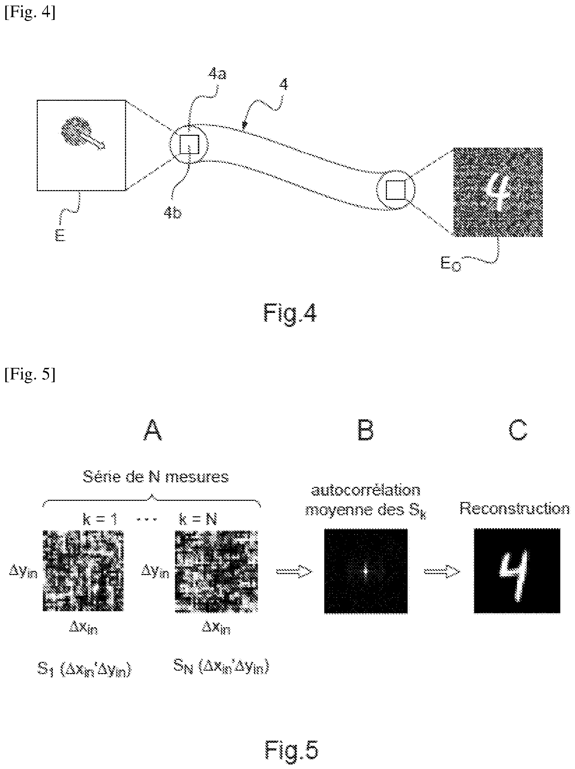

La [Fig.4] illustre schématiquement la fibre optique multimode à section carrée 4, comprenant une gaine cylindrique 4a recouvrant une âme 4b de section carrée.

Typiquement, la fibre optique multimode à section carrée 4 est souple, l'âme ayant des dimensions comprises entre 10 $\mu$m*10 $\mu$m et 1 mm*1 mm, de façon préférée comprises entre 50 $\mu$m*50 $\mu$m et 200 $\mu$m*200 $\mu$m, de façon davantage préférée comprise entre 100 $\mu$m*100 $\mu$m et 150 $\mu$m*150 $\mu$m.

On peut voir que l'on met en entrée de la fibre optique multimode à section carrée 4 un motif de speckle E que l'on translate sur tout ou partie de l'entrée de la fibre optique multimode à section carrée 4, générant en sortie de la fibre optique multimode à section carrée 4 un éclairage $E_O$ sur l'objet à imager.

Le procédé selon l'invention va maintenant être décrit plus en détail en liaison avec la [Fig.5].

Dans une première étape représentée en A sur la [Fig.5], pour N mesures, N un entier naturel supérieur ou égal à 1, un motif d'éclairage est balayé sur la face d'entrée de la fibre optique multimode à section carrée.

On considère un champ électromagnétique donné correspondant à un motif d'éclairage, numéroté

$$E_k^{in}\left(x_{in},\ y_{in}\right)$$

, k = 1...N, correspondant à un motif d'intensité :

[Math.2]

$$I_k^{in}\left(x_{in},\ y_{in}\right) = \left|E_k^{in}\left(x_{in},\ y_{in}\right)\right|^2$$

$x_{in}$ et $y_{in}$ sont les coordonnées dans la face d'entrée de la fibre optique multimode à section carrée. La procédure de mesure consiste à effectuer un balayage bidimensionnel de ce champ dans le plan de la fibre optique multimode à section carrée, c'est à dire à éclairer
la face d'entrée de la fibre optique multimode à section carrée avec des motifs de la forme :

[Math.3]

$$E_k^{in}\left(x_{in},\ y_{in},\ \Delta x_{in},\ \Delta y_{in}\right) = E_k^{in}\left(x_{in} - \Delta x_{in},\ y_{in} - \Delta y_{in}\right)$$

L'ensemble de toutes les valeurs de $\Delta x_{in}$ et $\Delta y_{in}$ définit un balayage, pour un motif donné $E_k^{in}\left(x_{in},\ y_{in}\right)$. En pratique, l'étendue du balayage est rectangulaire, avec

[Math.4]

$$\Delta x_{in} \in \left[\ \Delta x_{in}^{min},\ \Delta x_{in}^{max}\ \right]$$

et

[Math.5]

$$\Delta y_{in} \in \left[ \; \Delta y_{in}^{min}, \quad \Delta y_{in}^{max} \right]$$

et peut correspondre à :

- balayer le motif sur l'ensemble de la surface d'entrée de la fibre optique multimode à section carrée ;
- balayer le motif uniquement sur une partie limitée de la surface d'entrée de la fibre optique multimode à section carrée.

Cette procédure est répétée pour un nombre N de motifs différents, différents au sens où on ne retrouve jamais les mêmes motifs dans deux procédures de balayages (soit parce que les motifs de base sont différents, soit parce qu'un même motif est balayé sur des étendues différentes).

En pratique, il y a plusieurs dispositifs optiques permettant de générer des motifs et de les balayer :

- utilisation d'un dispositif modulateur spatial de lumière (SLM, acronyme de l'anglais Spatial Light Modulator), qui permet à la fois de choisir un motif défini sur les pixels du SLM, puis de le translater dans le plan d'entrée de la fibre optique multimode à section carrée. Il est possible d'utiliser soit un SLM à base de cristaux liquide (LCOS SLM), permettant de moduler la phase d'une onde lumineuse sur chaque pixel, soit un SLM de type matrice de micro-miroirs (DMD) permettant de moduler l'amplitude sur chaque pixel ;
- utilisation d'un diffuseur optique pour générer un motif d'apparence aléatoire, de type tavelures (speckle). Une modification de la position du diffuseur par rapport au faisceau d'éclairage permet alors de générer plusieurs motifs k = [1, ...,N]. Ce motif peut alors ensuite être balayé dans le plan de la fibre optique multimode à section carrée en translatant l'ensemble faisceau d'éclairage et du diffuseur, soit à l'aide de platine(s) de translation, soit à l'aide de miroirs galvanométriques.

Dans une deuxième étape, le signal généré par l'objet à imager en sortie de la fibre optique multimode à section carrée est mesuré.

On note $I_k^{out}\left( x_{out}, y_{out}, \; \Delta x_{in}, \; \Delta y_{in} \right)$ l'intensité optique en sortie de la fibre correspondant au champ $E_k^{in}\left( x_{in}, y_{in}, \; \Delta x_{in}, \; \Delta y_{in} \right)$ en entrée, pour une position de balayage définie par $\Delta \mathbf{r}_{in} = (\Delta x_{in}, \Delta y_{in})$.

On note $S_k(\Delta x_{in}, \Delta y_{in})$ le signal mesuré pour chaque position $\Delta \mathbf{r}_{in} = (\Delta x_{in}, \Delta y_{in})$. On fait l'hypothèse pour la reconstruction ultérieure que ce signal mesuré en présence d'un objet $O(x_{out}, y_{out})$ est de la forme :

[Math.6]

$$S_k\left( \; \Delta x_{in}, \; \Delta y_{in} \right) = \iint I_k^{out}\left( x_{out}, y_{out}, \; \Delta x_{in}, \; \Delta y_{in} \right) O\left( x_{out}, y_{out} \right) \mathrm{d}x_{out} \mathrm{d}y_{out}$$

Cette hypothèse correspond à de nombreuses situations, incluant les situations suivantes :

- le signal correspond à la lumière réfléchie par un échantillon réfléchissant ;
- le signal correspond à l'intensité de fluorescence émise par un objet fluorescent ; ou
- le signal correspond à un signal photoacoustique.

On obtient donc à l'issue des mesures un ensemble de N tableaux de valeurs noté $S_k(\Delta x_{in}, \Delta y_{in})$, k = 1 ...N, valeurs mesurées pour chaque position du balayage, qui contiennent de l'information sur l'objet à imager à reconstruire.

Dans une troisième et dernière étape, l'objet à imager est reconstruit à partir des mesures précédentes.

Dans tout ce qui suit, l'opérateur d'autocorrélation spatiale d'une fonction f est définie par :

[Math.7]

$$[ f \otimes f ]( \mathbf{r} ) = \iint f\left( \mathbf{r}' \right) f\left( \mathbf{r}' + \mathbf{r} \right) \mathrm{d}^2 \Delta \mathbf{r}'$$

Pour chaque mesure $S_k(\Delta x_{in}, \Delta y_{in})$, on calcule l'autocorrélation spatiale :

$$[\text{Math.8}]$$

$$C_k^S = S_k \otimes S_k$$

afin de calculer pour finir l'autocorrélation moyennée pour tous les motifs d'illumination (représentée en B sur la [Fig.5]) :

$$[\text{Math.9}]$$

$$C_{mesure}^S \left( \Delta x_{in}, \Delta y_{in} \right) = \left< C_k^S \left( \Delta x_{in}, \Delta y_{in} \right) \right>_{k=1..N}$$

A ce stade, la procédure est identique à celle décrite dans la publication scientifique « Non-invasive imaging through opaque scattering layers » (Imagerie non invasive à travers des couches de diffusion opaques), Jacopo Bertolotti, Elbert G Van Putten, Christian Blum, Ad Lagendijk, Willem L Vos, and Allard P Mosk, Nature, 491(7423) :232-234, 2012. On peut montrer que l'autocorrélation moyenne du signal s'écrit en fonction de l'autocorrélation moyenne des motifs d'intensité en sortie :

$$[\text{Math.10}]$$

$$C_{theorie}^S \left( \Delta \mathbf{r}_{in} \right) = \iint C^I \left( \Delta \mathbf{r}_{in}, \Delta \mathbf{r}_{out} \right) C^O \left( \Delta \mathbf{r}_{out} \right) \mathrm{d}^2 \Delta \mathbf{r}_{out}$$

où $C^I = \left< I_k^{out} \otimes I_k^{out} \right>$ est l'autocorrélation moyenne des N motifs d'éclairage et $C^O(\mathbf{r}) = \left< O(\mathbf{r}) \otimes O(\mathbf{r}) \right>$ l'autocorrélation de l'objet.

C'est l'équation Math. 10 qui est à la base de la possibilité de reconstruire l'objet O($\mathbf{r}$) à partir des mesures (étape C sur la [Fig.5]), le procédé général que nous proposons correspondant en effet à trouver un objet O($\mathbf{r}$) qui vérifie l'équation Math. 10. Pour ce faire, nous décrivons ici au moins deux méthodes possibles, sans caractère exhaustif.

La première méthode est la méthode dite de résolution de problème inverse.

Pour une fibre optique multimode à section carrée, on peut en effet montrer que l'autocorrélation moyenne des motifs d'éclairage peut s'écrire de façon explicite sous la forme :

$$[\text{Math.11}]$$

$$C^I \left( \Delta \mathbf{r}_{out}; \Delta \mathbf{r}_{in} \right) = \frac{1}{16} \left| \sum_{\in = \{+, \mp, -, \pm\}} \mu \left( 2\pi \frac{NA}{\lambda} \| \Delta \mathbf{r}_{out}, \Delta \mathbf{r}_{in}^\in \| \right) \right|^2$$

Avec

$$[\text{Math.12}]$$

$$\mu(\theta) = \frac{2J_1(\theta)}{\theta}$$

$J_1$ étant la fonction de Bessel de premier ordre et

$$[\text{Math.13}]$$

$$\Delta \mathbf{r}_{in}^+ = + \Delta \mathbf{r}_{in}$$

[Math.14]

$$\Delta \mathbf{r}_{in}^{\mp} = \left( - \Delta x_{in}, \ \Delta y_{in} \right)$$

[Math.15]

$$\Delta \mathbf{r}_{in}^{-} = - \Delta \mathbf{r}_{in}$$

[Math.16]

$$\Delta \mathbf{r}_{in}^{\pm} = \left( \Delta x_{in}, \ - \Delta y_{in} \right)$$

Les quatre vecteurs $\Delta \mathbf{r}_{in}^{\in}$ aux quatre directions de l'effet mémoire observé dans le cas de la fibre carrée. On peut alors estimer l'objet en résolvant le problème inverse suivant.

[Math.17]

$$\hat{O}_{estimé}(\mathbf{r}) = argmin_{X} \| C_{mesure}^{S}(\Delta \mathbf{r}_{in}) - \iint C^{I}(\Delta \mathbf{r}_{in}, \ \Delta \mathbf{r}_{out}) C^{X}(\Delta \mathbf{r}_{out}) \mathrm{d}^2 \Delta \mathbf{r}_{out} \|$$

où $C^X = \langle X \otimes X \rangle$ est l'autocorrélation d'un objet test X. L'équation Math. 17 signifie que l'estimation de l'objet $\hat{O}_{estimé}$ fournie par la résolution du problème est l'objet X qui minimise l'écart entre le résultat de mesure $C_{mesure}^{S}(\Delta \mathbf{r}_{in})$ et le modèle

[Math.18]

$$C_{theorie}^{S}(\Delta \mathbf{r}_{in}) = \iint C^{I}(\Delta \mathbf{r}_{in}, \ \Delta \mathbf{r}_{out}) C^{X}(\Delta \mathbf{r}_{out}) \mathrm{d}^2 \Delta \mathbf{r}_{out}$$

Il existe de nombreuses méthodes classiques pour résoudre ce type problème inverse , par exemple la méthode d'optimisation Adam, référence Kingma, Diederik P., and Jimmy Ba. "Adam: A method for stochastic optimization" (Adam : une méthode d'optimisation stochastique) arXiv preprint arXiv:1412.6980 (2014).

La deuxième méthode est la méthode par réseau de neurones.

Si l'objet à reconstruire appartient à une classe d'objets bien définie, on peut alors utiliser un réseau de neurones entraîné pour passer d'une "image" définie par $C^S(\Delta \mathbf{r}_{in})$ à l'objet O(r). Contrairement à la méthode précédente, cette méthode ne requiert pas de connaître une expression de $C^I(\Delta \mathbf{r}_{in}, \Delta \mathbf{r}_{out})$, mais est par contre limitée à une classe d'objets bien définie sur laquelle on peut entraîner un réseau de neurones.

La méthode a été mise en œuvre avec un réseau de type U-NET : pour la phase d'entraînement, on donne au réseau des couples $\{C^S(\Delta \mathbf{r}_{in}); O(\mathbf{r})\}_{connu}$ pour lesquels à la fois $C^S(\Delta \mathbf{r}_{in})$ et $O(\mathbf{r})$ sont connus. Le réseau de neurones est ensuite capable de fournir une estimation d'un objet à partir de $C_{mesure}^{S}(\Delta \mathbf{r}_{in})$.

La structure du réseau U-NET utilisé et la méthode est par exemple décrite dans la publication scientifique « Compensating for visibility artefacts in photoacoustic imaging with a deep learning approach providing prediction uncertainties » (Compensation d'artéfacts de visibilité en imagerie photoacoustique avec une approche apprentissage profond fournissant des incertitudes de prédiction), Guillaume Godefroy, Bastien Arnal, Emmanuel Bossy, Photoacoustics, 21 :100218, 2021. 4.

## Revendications

1. Procédé d'imagerie d'un objet à imager comprenant les suivantes étapes:

- générer, par un dispositif d'éclairage (2), une série de motifs d'éclairage ;
- pour chaque motif d'éclairage, réaliser une phase de stimulation dans laquelle des translations du motif d'éclairage sont réalisées en entrée d'une fibre optique multimode à section carrée (4) au moyen d'au moins un dispositif de translation (3a) d'un système optique (3) disposé entre le dispositif d'éclairage (2) et l'entrée de la fibre optique multimode à section carrée (4), les translations étant réalisées dans un plan parallèle au plan d'entrée de la fibre optique multimode à section carrée (4) ;
- pour chaque phase de stimulation, réaliser une phase d'acquisition dans laquelle le signal généré par le motif d'éclairage sur l'objet à imager (O) placé à la sortie de la fibre optique multimode à section carrée (4) est mesuré par un dispositif de mesure de signal (5 ; 9) ; et
- reconstruire par un dispositif de calcul (6), au moyen du signal mesuré pour chacune des phases d'acquisition, l'objet à imager (O).

2. Procédé d'imagerie selon la revendication 1, **caractérisé par le fait que** l'au moins un dispositif de translation (3a) est l'un parmi un modulateur spatial de lumière à base de cristaux liquides, un modulateur spatial de lumière de type matrice de micro-miroirs, un diffuseur optique associé à une platine de translation, un diffuseur optique associé à des miroirs galvanométriques.

3. Procédé d'imagerie selon la revendication 1 ou la revendication 2, **caractérisé par le fait que** le dispositif de mesure de signal (5) est un capteur optique de mesure du signal optique réfléchi par l'objet à imager associé à une fibre optique de détection placée entre l'objet à imager et le capteur optique, la fibre optique de détection étant l'une parmi la fibre optique multimode à section carrée (4) et une fibre optique auxiliaire (7).

4. Procédé d'imagerie selon la revendication 1 ou la revendication 2, **caractérisé par le fait que** le dispositif de mesure de signal est un hydrophone (9) à fibre optique (8) mesurant le signal photoacoustique de l'objet à imager (O).

5. Procédé d'imagerie selon la revendication 1 ou la revendication 2, **caractérisé par le fait que** l'objet à imager (O) est fluorescent et que le dispositif de mesure de signal (5) est un capteur optique de mesure du signal de fluorescence émis par l'objet à imager associé à une fibre optique de détection placée entre l'objet à imager et le capteur optique, la fibre optique de détection étant l'une parmi la fibre optique multimode à section carrée (4) et une fibre optique auxiliaire (7).

6. Procédé d'imagerie selon l'une quelconque des revendications 1 à 5, **caractérisé par le fait que**, dans chaque phase de stimulation, les translations sont réalisées dans tout ou partie du plan d'entrée de la fibre optique multimode à section carrée (4).

7. Procédé d'imagerie selon l'une quelconque des revendications 1 à 6, **caractérisé par le fait que** le dispositif d'éclairage (2) est une source de lumière cohérente.

8. Procédé d'imagerie selon l'une quelconque des revendications 1 à 7, **caractérisé par le fait que** la reconstruction de l'objet à imager (O) par le dispositif de calcul (6) comprend une première étape comprenant, pour chaque phase d'acquisition, le calcul de l'autocorrélation spatiale du signal mesuré puis le calcul de l'autocorrélation moyennée pour tous les motifs d'éclairage, et une seconde étape de déduction de l'objet à imager (O) à partir de l'autocorrélation moyennée pour tous les motifs d'éclairage obtenue dans la première étape.

9. Procédé d'imagerie selon la revendication 8, **caractérisé par le fait que** l'objet à imager (O) est déduit de l'autocorrélation moyennée pour tous les motifs d'éclairage par résolution de l'équation suivante : $\hat{O}_{estimé}(\mathbf{r}) =$

$$argmin_X \| C^S_{mesure}(\Delta\mathbf{r}_{in}) - \iint C^I(\Delta\mathbf{r}_{in}, \Delta\mathbf{r}_{out}) C^X(\Delta\mathbf{r}_{out}) \mathrm{d}^2\Delta\mathbf{r}_{out} \|$$ où $\Delta\mathbf{r}_{in} = (\Delta x_{in}, \Delta y_{in})$ définit une position de balayage dans la face d'entrée de la fibre optique multimode à section carrée avec $x_{in}$ et $y_{in}$ les coordonnées dans la face d'entrée de la fibre optique multimode à section carrée, $\Delta\mathbf{r}_{out}$ représente les coordonnées de la face de sortie de la fibre optique multimode à section carrée, $C^I$ est l'autocorrélation moyenne des N motifs d'éclairage, $C^X$ est l'autocorrélation d'un objet test X, $C^S_{mesure}$ est l'autocorrélation moyennée pour tous les motifs d'éclairage et $\hat{O}_{estimé}(\mathbf{r})$ est l'estimation obtenue de l'objet à imager (O).

10. Procédé d'imagerie selon la revendication 8, **caractérisé par le fait que** l'objet à imager (O) est déduit de l'autocorrélation moyennée pour tous les motifs d'éclairage par un réseau de neurones préalablement entraîné sur une classe d'objets définie, le réseau de neurones fournissant une estimation de l'objet à imager à partir de l'autocorrélation moyennée, par comparaison de l'autocorrélation moyennée connue par le réseau de neurones sur les objets de la classe définie à l'autocorrélation moyennée calculée pour l'objet à image.

11. Procédé d'imagerie selon l'une quelconque des revendications 1 à 10, **caractérisé par le fait que** la fibre optique multimode à section carrée (4) est souple.

12. Endoscope (1 ; 10 ; 20) pour l'imagerie d'un objet à imager (O), comprenant un dispositif d'éclairage (2), un système optique (3), au moins une fibre optique multimode à section carrée (4), le système optique (3) couplant optiquement le dispositif d'éclairage (2) à l'entrée de l'au moins une fibre optique multimode à section carrée (4), le système optique (3) comprenant un dispositif de translation (3a) pour translater un motif d'éclairage généré par le dispositif d'éclairage (2) sur tout ou partie du plan d'entrée de l'au moins une fibre optique multimode à section carrée (4), le dispositif de translation (3a) étant configuré pour translater le motif d'éclairage dans un plan parallèle au plan d'entrée de l'au moins fibre optique multimode à section carrée (4), l'objet à imager (O) étant configuré pour être placé en sortie de l'au moins une fibre optique multimode à section carrée (4), un dispositif de mesure de signal (5, 9) généré par l'objet à imager (O) et un dispositif de calcul (6) pour mettre en œuvre le procédé selon l'une quelconque des revendications 1 à 11.

13. Endoscope (1 ; 10 ; 20) selon la revendication 12, **caractérisé par le fait que** le dispositif de translation (3a) étant l'un parmi un modulateur spatial de lumière à base de cristaux liquides, un modulateur spatial de lumière de type matrice de micro-miroirs, un diffuseur optique associé à une platine de translation, un diffuseur optique associé à des miroirs galvanométriques.

14. Endoscope (1 ; 10) selon la revendication 12 ou la revendication 13, **caractérisé par le fait que** le dispositif de mesure de signal (5) est constitué par au moins l'un parmi :

- l'au moins une fibre optique multimode à section carrée (4) associée à un capteur optique en entrée de la fibre optique multimode à section carrée (4) pour capter un signal optique réfléchi par l'objet à imager (O),
- au moins une fibre optique auxiliaire (7) associée à un capteur optique du côté entrée de la fibre optique multimode à section carrée (4) de l'endoscope (1 ; 10) pour capter un signal optique réfléchi par l'objet à imager (O),
- un hydrophone (9) à fibre optique (8) pour capter le signal photoacoustique de l'objet à imager (O).

15. Endoscope (1 ; 10) selon la revendication 12 ou la revendication 13, **caractérisé par le fait que** l'objet à imager (O) est fluorescent et que le dispositif de mesure de signal (5) est constitué par au moins l'un parmi :

- l'au moins une fibre optique multimode à section carrée (4) associée à un capteur optique en entrée de la fibre optique multimode à section carrée (4) pour capter un signal de fluorescence de l'objet à imager (O),
- au moins une fibre optique auxiliaire (7) associée à un capteur du côté entrée de la fibre optique multimode à section carrée (4) de l'endoscope (1 ; 10) pour capter un signal de fluorescence de l'objet à imager (O).

16. Endoscope (1 ; 10 ; 20) selon l'une quelconque des revendications 12 à 15, **caractérisé par le fait que** l'au moins une fibre multimode à section carrée (4) est souple.

17. Produit programme d'ordinateur, **caractérisé par le fait qu'**il comprend des instructions qui, lorsqu'elles sont chargées et exécutées sur un dispositif de calcul d'un endoscope selon l'une quelconque des revendications 12 à 16, permettent de mettre en œuvre le procédé selon l'une quelconque des revendications 1 à 11.

**Patentansprüche**

1. Bildgebungsverfahren für ein abzubildendes Objekt, umfassend die folgenden Schritte:

- Erzeugen einer Reihe von Beleuchtungsmustern durch eine Beleuchtungsvorrichtung (2);
- für jedes Beleuchtungsmuster Durchführen einer Stimulationsphase, in der Übertragungen des Beleuchtungsmusters an dem Eingang einer optischen Multimode-Faser mit quadratischem Querschnitt (4) mittels mindestens einer Übertragungsvorrichtung (3a) eines optischen Systems (3) durchgeführt werden, das zwischen der Beleuchtungsvorrichtung (2) und dem Eingang der optischen Multimode-Faser mit quadratischem Querschnitt (4) angeordnet ist, wobei die Übertragungen in einer Ebene parallel zu der Eingangsebene der optischen Multimode-Faser mit quadratischem Querschnitt (4) durchgeführt werden;
- für jede Stimulationsphase Durchführen einer Erfassungsphase, in der das durch das Beleuchtungsmuster auf dem abzubildenden Objekt (O), das an dem Ausgang der optischen Multimode-Faser mit quadratischem Querschnitt (4) positioniert ist, erzeugte Signal durch eine Signalmesseinrichtung (5; 9) gemessen wird; und
- Rekonstruieren des abzubildenden Objekts (O) durch eine Rechenvorrichtung (6) anhand des für jede der Erfassungsphasen gemessenen Signals.

2. Bildgebungsverfahren nach Anspruch 1, **dadurch gekennzeichnet, dass** die mindestens eine Übertragungsvorrichtung (3a) eine der Folgenden ist, ein räumlicher Lichtmodulator auf Flüssigkristallbasis, ein räumlicher Lichtmodulator vom Typ einer Mikrospiegelmatrix, ein optischer Diffusor, der einer Übertragungsplatte zugeordnet ist, ein optischer Diffusor, der galvanometrischen Spiegeln zugeordnet ist.

3. Bildgebungsverfahren nach Anspruch 1 oder Anspruch 2, **dadurch gekennzeichnet, dass** die Signalmessvorrichtung (5) ein optischer Sensor zum Messen des von dem abzubildenden Objekt reflektierten optischen Signals ist, der einer optischen Detektionsfaser zugeordnet ist, die zwischen dem abzubildenden Objekt und dem optischen Sensor positioniert ist, wobei die optische Detektionsfaser eine von der optischen Multimode-Faser mit quadratischem Querschnitt (4) und einer optischen Zusatzfaser (7) ist.

4. Bildgebungsverfahren nach Anspruch 1 oder Anspruch 2, **dadurch gekennzeichnet, dass** die Signalmesseinrichtung ein Hydrophon (9) mit optischer Faser (8) ist, das das photoakustische Signal des abzubildenden Objekts (O) misst.

5. Bildgebungsverfahren nach Anspruch 1 oder Anspruch 2, **dadurch gekennzeichnet, dass** das abzubildende Objekt (O) fluoreszierend ist und dass die Signalmessvorrichtung (5) ein optischer Sensor zum Messen des von dem abzubildenden Objekt emittierten Fluoreszenzsignals ist, der einer optischen Detektionsfaser zugeordnet ist, die zwischen dem abzubildenden Objekt und dem optischen Sensor positioniert ist, wobei die optische Detektionsfaser eine von der optischen Multimode-Faser mit quadratischem Querschnitt (4) und einer optischen Zusatzfaser (7) ist.

6. Bildgebungsverfahren nach einem der Ansprüche 1 bis 5, **dadurch gekennzeichnet, dass** in jeder Stimulationsphase die Übertragungen in der gesamten oder einem Teil der Eingangsebene der optischen Multimode-Faser mit quadratischem Querschnitt (4) ausgeführt werden.

7. Bildgebungsverfahren nach einem der Ansprüche 1 bis 6, **dadurch gekennzeichnet, dass** die Beleuchtungsvorrichtung (2) eine kohärente Lichtquelle ist.

8. Bildgebungsverfahren nach einem der Ansprüche 1 bis 7, **dadurch gekennzeichnet, dass** die Rekonstruktion des abzubildenden Objekts (O) durch die Rechenvorrichtung (6) einen ersten Schritt umfasst, der für jede Erfassungsphase die Berechnung der räumlichen Autokorrelation des gemessenen Signals und dann die Berechnung der für alle Beleuchtungsmuster gemittelten Autokorrelation umfasst, und einen zweiten Schritt der Ableitung des abzubildenden Objekts (O) aus der für alle Beleuchtungsmuster gemittelten Autokorrelation, die im ersten Schritt erhalten wurde.

9. Bildgebungsverfahren nach Anspruch 8, **dadurch gekennzeichnet, dass** das abzubildende Objekt (O) aus der über alle Beleuchtungsmuster gemittelten Autokorrelation durch Lösen der folgenden Gleichung abgeleitet wird:

$$\hat{O}_{gesch\ddot{a}tzt}(\mathrm{r}) = ar\,gmin_X \left\| C^S_{Messung}(\Delta \mathrm{r}_{in}) - \iint C^I(\Delta \mathrm{r}_{in}, \Delta \mathrm{r}_{out}) C^X(\Delta \mathrm{r}_{out}) \mathrm{d}2\Delta \mathrm{r}_{out} \right\|$$

, wobei $\Delta \mathrm{r}_{in} = (\Delta x_{in},\, \Delta y_{in})$ eine Abtastposition in der Eingangsfläche der optischen Multimode-Faser mit quadratischem Querschnitt mit $x_{in}$ und $y_{in}$ den Koordinaten in der Eingangsfläche der optischen Multimode-Faser mit quadratischem Querschnitt definiert, $\Delta \mathrm{r}_{out}$ die Koordinaten der Ausgangsfläche der optischen Multimode-Faser mit quadratischem Querschnitt darstellt, $C^I$ die mittlere Autokorrelation der N Beleuchtungsmuster ist, $C^X$ die Autokorrelation eines Testobjekts X ist, $C^S_{Messung}$ die für alle Beleuchtungsmuster gemittelte Autokorrelation ist und $\hat{O}_{gesch\ddot{a}tzt}(\mathrm{r})$ die erhaltene Schätzung des abzubildenden Objekts (O) ist.

10. Bildgebungsverfahren nach Anspruch 8, **dadurch gekennzeichnet, dass** das abzubildende Objekt (O) aus der über alle Beleuchtungsmuster gemittelten Autokorrelation durch ein zuvor auf eine definierte Objektklasse trainiertes neuronales Netz abgeleitet wird, wobei das neuronale Netz aus der gemittelten Autokorrelation eine Schätzung des abzubildenden Objekts bereitstellt, durch Vergleich der gemittelten Autokorrelation, die dem neuronalen Netzwerk für die Objekte der definierten Klasse bekannt ist, mit der gemittelten Autokorrelation, die für das abzubildende Objekt berechnet wurde.

11. Bildgebungsverfahren nach einem der Ansprüche 1 bis 10, **dadurch gekennzeichnet, dass** die optische Multimode-Faser mit quadratischem Querschnitt (4) flexibel ist.

12. Endoskop (1; 10; 20) zur Bildgebung eines abzubildenden Objekts (O), umfassend eine Beleuchtungsvorrichtung (2), ein optisches System (3), mindestens eine optische Multimode-Faser mit quadratischem Querschnitt (4), wobei das optische System (3) die Beleuchtungsvorrichtung (2) optisch mit dem Eingang der mindestens einen optischen Multimode-Faser mit quadratischem Querschnitt (4) koppelt, wobei das optische System (3) eine Übertragungsvorrichtung (3a) umfasst, um ein von der Beleuchtungsvorrichtung (2) erzeugtes Beleuchtungsmuster auf die gesamte oder einen Teil der Eingangsebene der mindestens einen optischen Multimode-Faser mit quadratischem Querschnitt (4) zu übertragen, wobei die Übertragungsvorrichtung (3a) so konfiguriert ist, dass sie das Beleuchtungsmuster in eine Ebene parallel zu der Eingangsebene der mindestens einen optischen Multimode-Faser mit quadratischem Querschnitt (4) überträgt, wobei das abzubildende Objekt (O) so konfiguriert ist, dass es an dem Ausgang der mindestens einen optischen Multimode-Faser mit quadratischem Querschnitt (4) positioniert wird, eine Vorrichtung zur Messung des von dem abzubildenden Objekts (O) erzeugten Signals (5, 9) und eine Rechenvorrichtung (6) zum Implementieren des Verfahrens nach einem der Ansprüche 1 bis 11.

13. Endoskop (1; 10; 20) nach Anspruch 12, **dadurch gekennzeichnet, dass** die Übertragungsvorrichtung (3a) entweder ein räumlicher Lichtmodulator auf Flüssigkristallbasis, ein räumlicher Lichtmodulator vom Typ einer Mikrospiegelmatrix, ein einer Übertragungsplatte zugeordneter optische Diffusor oder ein galvanometrischen Spiegeln zugeordneter optischer Diffusor ist.

14. Endoskop (1; 10) nach Anspruch 12 oder Anspruch 13, **dadurch gekennzeichnet, dass** die Signalmesseinrichtung (5) aus mindestens einem der folgenden Elemente besteht:

- der mindestens einen optischen Multimode-Faser mit quadratischem Querschnitt (4), die einem optischen Sensor am Eingang der optischen Multimode-Faser mit quadratischem Querschnitt (4) zugeordnet ist, um ein von dem abzubildenden Objekt (O) reflektiertes optisches Signal aufzunehmen,
- mindestens einer optischen Zusatzfaser (7), die einem optischen Sensor auf der Eingangsseite der optischen Multimode-Faser mit quadratischem Querschnitt (4) des Endoskops (1; 10) zugeordnet ist, um ein von dem abzubildenden Objekt (O) reflektiertes optisches Signal aufzunehmen,
- einem Hydrophon (9) mit optischer Faser (8) zum Aufnehmen des photoakustischen Signals des abzubildenden Objekts (O).

15. Endoskop (1; 10) nach Anspruch 12 oder Anspruch 13, **dadurch gekennzeichnet, dass** das abzubildende Objekt (O) fluoreszierend ist und dass die Signalmesseinrichtung (5) aus mindestens einem der folgenden Elemente besteht:

- der mindestens einen optischen Multimode-Faser mit quadratischem Querschnitt (4), die einem optischen Sensor am Eingang der optischen Multimode-Faser mit quadratischem Querschnitt (4) zugeordnet ist, um ein Fluoreszenzsignal des abzubildenden Objekts (O) aufzunehmen,
- mindestens einer optischen Zusatzfaser (7), die einem Sensor auf der Eingangsseite der optischen Multimode-Faser mit quadratischem Querschnitt (4) des Endoskops (1; 10) zugeordnet ist, um ein Fluoreszenzsignal des abzubildenden Objekts (O) aufzunehmen.

16. Endoskop (1; 10; 20) nach einem der Ansprüche 12 bis 15, **dadurch gekennzeichnet, dass** die mindestens eine Multimode-Faser mit quadratischem Querschnitt (4) flexibel ist.

17. Computerprogrammprodukt, **dadurch gekennzeichnet, dass** es Anweisungen umfasst, die, wenn sie auf eine Rechenvorrichtung eines Endoskops nach einem der Ansprüche 12 bis 16 geladen und dort ausgeführt werden, das Implementieren des Verfahrens nach einem der Ansprüche 1 bis 11 ermöglichen.

**Claims**

1. Imaging process for imaging an object to be imaged (O), comprising the following steps:

   - generating, by a lighting device (2), a series of lighting patterns;
   - for each lighting pattern, performing a stimulation phase in which translations of the lighting pattern are performed at the input of an optical multimode fiber with a square cross-section (4) using at least one translation device (3a) of an optical system (3) arranged between the lighting device (2) and the input of the optical multimode fiber with a square cross-section (4), the translations being performed in a plane parallel to the input plane of the optical multimode fiber with a square cross-section (4);
   - for each stimulation phase, performing an acquisition phase in which the signal generated by the lighting pattern on the object to be imaged (O) positioned at the output of the optical multimode fiber with a square cross-section (4) is measured by a signal measuring device (5; 9); and
   - reconstructing the object to be imaged (O) by a computing device (6) based on the signal measured for each of the acquisition phases.

2. Imaging process according to claim 1, **characterized by** the at least one translation device (3a) is one of the following: a liquid crystal-based spatial light modulator, a micro-mirror array type spatial light modulator, an optical diffuser associated with a translation stage, and an optical diffuser associated with galvanometer mirrors.

3. Imaging process according to claim 1 or claim 2, **characterized by** the signal measuring device (5) is an optical sensor for measuring the optical signal reflected by the object to be imaged, associated with a detection optical fiber placed between the object to be imaged and the optical sensor, the detection optical fiber being one of the optical multimode fibers with a square cross-section (4) and an auxiliary optical fiber (7).

4. Imaging process according to claim 1 or claim 2, **characterized by** the signal measuring device is a fiber-optic (8) hydrophone (9) measuring the photoacoustic signal of the object to be imaged (O).

5. Imaging process as claimed in claim 1 or claim 2, **characterized by** the object to be imaged (O) is fluorescent and the signal measuring device (5) is an optical sensor for measuring the fluorescence signal emitted by the object to be imaged, associated with a detection optical fiber placed between the object to be imaged and the optical sensor, the detection optical fiber being one of the optical multimode fibers with a square cross-section (4) and an auxiliary optical fiber (7).

6. Imaging process according to any one of claims 1 to 5, **characterized by**, in each stimulation phase, the translations are carried out in all or part of the input plane of the optical multimode fibers with a square cross-section (4).

7. Imaging process according to any one of claims 1 to 6, **characterized by** the lighting device (2) is a coherent light source.

8. Imaging process according to any one of claims 1 to 7, **characterized by** the reconstruction of the object to be imaged (O) by the calculation device (6) comprises a first step comprising, for each acquisition phase, the calculation of the spatial autocorrelation of the measured signal then the calculation of the averaged autocorrelation for all the lighting patterns, and a second step of deducing the object to be imaged (O) from the averaged autocorrelation for all the lighting patterns obtained in the first step.

9. Imaging process according to claim 8, **characterized by** the object to be imaged (O) is deduced from the averaged autocorrelation for all the lighting patterns by solving the following equation:

$$\hat{O}_{estim}(\mathbf{r}) = argmin_X \parallel C^S_{mesure}(\Delta \mathbf{r}_{in}) - \iint C^I(\Delta \mathbf{r}_{in}, \Delta \mathbf{r}_{out}) C^X(\Delta \mathbf{r}_{out}) \mathrm{d}^2 \Delta \mathbf{r}_{out} \parallel,$$

where $\Delta\mathbf{r}_{in} = (\Delta x_{in}, \Delta y_{in})$ defines a scanning position in the input face of the square-section multimode optical fiber with $x_{in}$ and $y_{in}$ the coordinates in the input face of the square-section multimode optical fiber, $\Delta\mathbf{r}_{out}$ represents the coordinates of the output face of the square-section multimode optical fiber, $C^I$ is the averaged autocorrelation of the N lighting patterns, $C^X$ is the autocorrelation of a test object X, $C^S_{mesure}$ is the averaged autocorrelation for all the

lighting patterns and $\hat{O}_{estim}(\mathbf{r})$ is the obtained estimate of the object to be imaged (O).

10. Imaging process according to claim 8, **characterized by** the object to be imaged (O) is deduced from the averaged autocorrelation for all the lighting patterns by a neural network previously trained on a defined class of objects, the neural network providing an estimate of the object to be imaged from the averaged autocorrelation, by comparing the averaged autocorrelation known by the neural network on the objects of the defined class with the averaged autocorrelation calculated for the object to be imaged.

11. Imaging process according to any one of claims 1 to 10, **characterized by** the square-section multimode optical fiber (4) is flexible.

12. Endoscope (1; 10; 20) for imaging an object to be imaged (O), comprising a lighting device (2), an optical system (3), at least one multimode optical fiber with a square section (4), the optical system (3) optically coupling the lighting device (2) to the input of the at least one multimode optical fiber with a square section (4), the optical system (3) comprising a translation device (3a) for translating a lighting pattern generated by the lighting device (2) over all or part of the input plane of the at least one multimode optical fiber with a square section (4), the translation device (3a) being configured to translate the lighting pattern in a plane parallel to the input plane of the at least one multimode optical fiber with a square section (4), the object to be imaged (O) being configured to be placed at the output of the at least one multimode optical fiber with a square section (4), a signal measuring device (5, 9) generated by the object to be imaged (O) and a computing device (6) for implementing the method according to any one of claims 1 to 11.

13. Endoscope (1; 10; 20) according to claim 12, **characterized by** the translation device (3a) is one of a liquid crystal-based spatial light modulator, a micro-mirror matrix type spatial light modulator, an optical diffuser associated with a translation stage, an optical diffuser associated with galvanometric mirrors.

14. Endoscope (1; 10) according to claim 12 or claim 13, **characterized by** the signal measuring device (5) is constituted by at least one of:

   - at least one multimode optical fiber with square section (4) associated with an optical sensor at the input of the multimode optical fiber with square section (4) for capturing an optical signal reflected by the object to be imaged (O),
   - at least one auxiliary optical fiber (7) associated with an optical sensor on the input side of the multimode optical fiber with square section (4) of the endoscope (1; 10) for capturing an optical signal reflected by the object to be imaged (O),
   - a hydrophone (9) with optical fiber (8) for capturing the photoacoustic signal of the object to be imaged (O).

15. Endoscope (1; 10) according to claim 12 or claim 13, **characterized by** the object to be imaged (O) is fluorescent and that the signal measuring device (5) is constituted by at least one of:

   - at least one multimode optical fiber with square section (4) associated with an optical sensor at the input of the multimode optical fiber with square section (4) to capture a fluorescence signal from the object to be imaged (O),
   - at least one auxiliary optical fiber (7) associated with a sensor on the input side of the multimode optical fiber with square section (4) of the endoscope (1; 10) to capture a fluorescence signal from the object to be imaged (O).

16. Endoscope (1; 10; 20) according to any one of claims 12 to 15, **characterized by** the at least one multimode fiber with square section (4) is flexible.

17. Computer program product, **characterized by** it comprises instructions which, when loaded and executed on a computing device of an endoscope according to any one of claims 12 to 16, make it possible to implement the method according to any one of claims 1 to 11.

[Fig. 1]

Fig.1

[Fig. 2]

Fig.2

[Fig. 3]

Fig.3

[Fig. 4]

Fig.4

[Fig. 5]

Fig.5

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 20150015879 A1
- US 5956447 A
- US 10254534 B2
- US 10809750 B2
- US 10398294 B2

**Littérature non-brevet citée dans la description**

- **GUILLAUME GODEFROY** ; **BASTIEN ARNAL** ; **EMMANUEL BOSSY**. Compensating for visibility artefacts in photoacoustic imaging with a deep learning approach providing prediction uncertainties » (Compensation d'artéfacts de visibilité en imagerie photoacoustique avec une approche apprentissage profond fournissant des incertitudes de prédiction. *Photoacoustics*, April 2021, vol. 21, 100218
- **JACOPO BERTOLOTTI** ; **ELBERT G VAN PUTTEN** ; **CHRISTIAN BLUM** ; **AD LAGENDIJK** ; **WILLEM L VOS** ; **ALLARD P MOSK**. Non-invasive imaging through opaque scattering layers » (Imagerie non invasive à travers des couches de diffusion opaques). *Nature*, 2012, vol. 491 (7423), 232-234
- **KINGMA, DIEDERIK P.** ; **JIMMY BA**. Adam: A method for stochastic optimization'' (Adam : une méthode d'optimisation stochastique). *ar-Xiv:1412.6980*, 2014
- **GUILLAUME GODEFROY** ; **BASTIEN ARNAL** ; **EMMANUEL BOSSY**. Compensating for visibility artefacts in photoacoustic imaging with a deep learning approach providing prediction uncertainties » (Compensation d'artéfacts de visibilité en imagerie photoacoustique avec une approche apprentissage profond fournissant des incertitudes de prédiction). *Photoacoustics*, April 2021, vol. 21, 100218